# EUROPEAN PATENT SPECIFICATION

(11) **EP 3 318 323 B1**
(45) Date of publication and mention of the grant of the patent: **12.08.2020**
(21) Application number: 16753471.8
(22) Date of filing: 05.07.2016
(51) Int. Cl.: B01J 13/12, B01J 13/16, A01N 25/28, A61K 9/50, A61K 51/12, C11D 3/50

(54) **MICRO- OR NANOCAPSULES HAVING PHOTOCATALYTIC PROPERTIES FOR CONTROLLED RELEASE OF DIFFUSING AGENTS AND RESPECTIVE METHODS OF OBTAINMENT**
MIKRO- ODER NANOKAPSELN MIT PHOTOKATALYTISCHEN EIGENSCHAFTEN ZUR GESTEUERTEN FREISETZUNG VON DIFFUSIONSMITTELN UND ZUGEHÖRIGES VERFAHREN ZUR HERSTELLUNG
MICROCAPSULES OU NANOCAPSULES À PROPRIÉTÉS PHOTOCATALYTIQUES POUR LA LIBÉRATION CONTRÔLÉE D'AGENTS DIFFUSEURS ET PROCÉDÉ D'OBTENTION RESPECTIF

(30) Priority: 05.07.2015 PT 2015108665
(43) Date of publication of application: 09.05.2018
(73) Proprietor: Universidade do Minho, 4704-553 Braga (PT)
(72) Inventor: MACEDO TAVARES, Carlos José, 4804-533 Guimarães (PT); GOUVEIA MARQUES, Juliana Filipa, 4804-533 Guimarães (PT)
(74) Representative: Patentree
(86) International application number: PCT/IB2016/054027
(87) International publication number: WO 2017/006247

(56) References cited:
- WO-A2-2011/012935
- US-A1- 2010 054 988
- LI Z ET AL: "Preparation of chitosan-sodium alginate microcapsules containing ZnS nanoparticles and its effect on the drug release", MATERIALS SCIENCE AND ENGINEERING C, ELSEVIER SCIENCE S.A, CH, vol. 29, no. 7, 31 August 2009 (2009-08-31), pages 2250-2253, XP026446101, ISSN: 0928-4931 [retrieved on 2009-05-22]

## Description

### Field of the invention

The present invention lies within the field of production of functional coatings for the controlled release of volatile agents. More specifically, it consists of capsules, in particular microcapsules or nanocapsules chemically functionalised with photocatalytic nanomaterials upon the internal or external surface of the wall of the capsule which, by solar action or artificial light having the same spectrum of electromagnetic radiation, release the diffusing/active agent, this being a vapour, liquid, or solid.

The applications include the pharmaceutical area, biotechnology, civil engineering, health, agrochemistry, automotive and foodstuffs.

### Background

The present invention consists of a technology of heterostructured materials having the ability to disperse, by solar activation, certain agents encapsulated in microcapsules or nanocapsules functionalised with photocatalytic nanomaterials. The photocatalytic nanomaterials may be nanostructures, such as nanotubes, nanoparticles, nanofibres or quantum dots, depending on the intended functionality. The agents or products to be released may be encapsulated in the polymeric microcapsules or nanocapsules, in a solid, liquid or vapour phase. By solar activation, or by other radiation having similar properties, preferably incorporating ultraviolet radiation, the photocatalytic nanomaterials, being semiconductors having a band gap of between 2.8 and 3.4 eV, will absorb this radiation and promote electronic transitions between the valence and the conduction bands, subsequently giving rise to mechanisms of oxidation/reduction (redox). These redox mechanisms initiate the degradation or rupture of the wall of the microcapsule, in this manner promoting the diffusion of the encapsulated agent. Several types of microcapsules already exist in the market releasing certain agents by direct diffusion through the wall of porous microcapsules or by mechanical action: friction, fissuring, crushing. However, in static substrates, wherein the mechanisms of mechanical action are not available, this technology solves that problem by activating the diffusion of the agents by light exposure.

Some examples of technologies using microcapsules are referred to in the literature, however in a very distinct manner.

The patent document WO2009/062516 describes panels having a coating constituted by several layers of nanoparticles deposited upon a surface. It furthermore adds that one of these layers may be of photocatalytic nanoparticles, or may also have layers having particles possessing antimicrobial or deodorising properties. More specifically, it relates to a self-cleaning surface for floors or panels of wood consisting essentially in the dispersion of photocatalytic nanoparticles within a binder polymeric matrix, for example a resin or a varnish, which can be applied, for example, upon the wooden floor. When the nanoparticles are in contact with moisture, they will convert this water into a hydrophilic film (wetting the surface) which, for example, by electrostatic repulsion, will make the dirt remain on the surface of this film of water, being easily removed. This technology facilitates cleaning and renders this film of water removable (dried) with greater facility.

The patent documents EP1531667 B2 and US6077522 A disclose porous microcapsules containing a biologically active material being sensitive to ultraviolet light. These capsules are prepared to contain an ultraviolet radiation protector for the biologically active material, selected from titanium dioxide, zinc oxide and mixtures thereof, suspended and completely dispersed in the liquid, and a dispersant serving to disperse the ultraviolet radiation protector in the organic liquid, and to retain it in the aforementioned liquid, but which does allow it to be extracted by diffusion, for example into water. This process is not related to the effect of controlled release of a substance by the direct action of light or by photocatalytic processes induced by photocatalytic materials physically bound to microcapsules containing a volatile agent to be diffused.

The patent document US2009010977 A1 describes the synthesis of nanocapsules with permethrin, without any nanomaterial or principle. It discloses the use of solar energy solely to check the level of prolonged activity of nanocapsules, which rupture under friction (mechanical action).

The patent document WO2007/051198 describes the synthesis of microcapsules, which slowly diffuse certain agents by virtue of utilising photosensitive polymers in the formation thereof. The structure of the microcapsule wall is functionalised by means of catalysts, which initiate the degradation thereof by a process of solar sensitisation, without relying on photocatalytic nanomaterials, as in the case of the present technology. The presence of the photocatalytic nanomaterials makes the release more efficient and controlled in a better manner through the solar exposure.

The underlying invention in the patent document GB1513614 A relates to a composition of a microencapsulated auxiliary agent, destined to be released into the soil by a diffusion process from within a porous polymeric microcapsule, aided by the drainage of water. This is suitable for agrochemical products, pharmaceutical products, inks and dyes, such as an active component contained within a wall casing of the microcapsule. The porosity of the wall of the microcapsule is designed to deliver the slow release. This process is not related to photocatalysis, nor to the effect of the controlled release of a substance by the direct action of light or by photocatalytic processes induced by photocatalytic materials physically bound to microcapsules containing an agent to be diffused. Hence, they cannot be used, for example, in static substrates exposed to the sun.

The document JP6228882 A discloses an insect proof textile structure having slow release of an insecticide. The insecticide either is encapsulated in porous microcapsules or is adsorbed onto the textile mesh, not having an effect which may be activated and controlled by exposure to light. This technology does not use the principle of photocatalysis, nor the effect of controlled release of a substance by the direct action of light.

The documents EP0376385 A2 and US7786027 describe processes for the synthesis of microcapsules containing a detergent/softener, without alluding to a photocatalytic or solar activation process. The detergent/softener is diffused through the open pores intrinsic to the microcapsule.

Document JP2004188325 A discloses the use of porous microparticles for ammonia degradation. It is not a matter of microcapsules functionalised with photocatalytic nanomaterials for diffusing a specific agent, it is simply a matter of a porous microparticle having on the surface a dispersion of photocatalytic particles. The aforementioned particle solely acts as substrate in order for these particles to decompose when they are in contact with ammonia. The microparticles are porous and, as such, they do not render possible the controlled release of any internal agent.

The document WO2009048186 A1 discloses nanoparticles of titanium dioxide enveloped in a metal nucleus. This nucleus is not a microcapsule, solely a substrate. There is no mechanism of controlled release from this nucleus. The nucleus provides a larger surface area for the particle such that it may undertake redox processes to decontaminate pollutants.

The document WO2004022841 A1 discloses a system wherein, in one of the particular embodiments, photocatalytic nanoparticles are dispersed in a binder sublayer of a varnish, for example for application on wooden floors. In a particular embodiment, there may also be a dispersion of microcapsules in this sublayer of this varnish, containing a deodorising agent, however there being binding apparent between the microcapsules and nanoparticles. The object is to produce a hydrophilic surface permitting a better cleaning of the surface thereof and releasing a deodorising agent or an antibacterial agent by mechanical action. There is no reference to the release of any substance by solar activation. However, the document divulges that, by mechanical action, the capsules, or simply the agent itself lodged in previously generated microfissures, may be diffused following the superficial layer having been scratched, trodden upon, crushed, or suffering another type of mechanical action rendering possible the release of the product. Following the exhaustion of the active ingredient the material cannot be regenerated.

The document WO2011012935 discloses a coating heterostructured in layers, characterised in that it comprises a substrate; photocatalytic material in the form of solid thin film; polymeric nano- or microcapsules having an encapsulated diffusing agent. This technology presupposes the existence of a photocatalytic base material, previously deposited on the substrate, which in contact with the wall of the microcapsule initiates the redox process to release the diffusing agent. This technology differs from the technology of the present invention by virtue of the fact that in the present invention the said photocatalytic substrate or substrate is not necessary since the microcapsule wall is functionalised with the photocatalytic materials in the process of the synthesis thereof. Additionally, the microcapsules are of different origin. The regeneration of the active surface signifies that a photocatalytic coating has been previously deposited upon the surface, such that it may be subsequently regenerated, for example by means of the spraying thereof with an aerosol containing microcapsules having a particular volatile agent.

Document WO2011012935 discloses a heterostructured coating with functional characteristics that enables the controlled release of volatile agents. Document WO2011012935 discloses a photocatalytic film deposited directly onto a substrate, where the nano or microcapsules only contact with the photocatalytic material surface on the point of contact.

The technology of the present invention has advantages in relation to the other technologies referred to in the literature, by virtue of the fact that it may be applied in untreated static substrates, simply by attachment to the substrate, which may be clothing, a tent, mosquito net, curtain, awning, or any other substrate or structure having direct solar exposure or to equivalent ultraviolet radiation, through spraying, or deposition, of the system of functionalised microcapsules having the photocatalytic nanomaterials and certain encapsulated agents. In order for the diffusing agent to be released, in a controlled manner, it does not require a mechanical initiation rupturing the wall of the microcapsule, by virtue of the fact that this activation will be realised solely by mechanisms of oxidation/reduction associated with the intrinsic process of the photocatalysis of the nanomaterials which are functionalised on the exterior wall of the microcapsule.

These facts are presented to illustrate the problem solved by the present invention.

### General Description

The present invention is characterised by using micro- and nanofunctional materials, capable of promoting the controlled release of a diffusing agent.

The present invention describes nanocapsules and microcapsules comprising, preferably, a diameter from 100 -1000 nm and 1 - 500 µm, respectively, and through solar action or artificial light having a similar spectrum of electromagnetic radiation there is promoted a redox reaction resulting in the dissociation or rupture of the wall of the capsule and subsequent release of the diffusing agent which may be solid, liquid or vapour. This technology takes advantage of the photocatalytic and semiconductor effect already established for titanium dioxide for use as an active surface, promoting the controlled release of a given diffusing agent from within the polymeric micro- or nanocapsules, whether, inter alia, insecticides, larvicides, repellents, pesticides, phytonutrients, fragrances, additives for paint or varnish, or deodorants.

In this solution, nanomaterials based on titanium dioxide, such as nanoparticles having a diameter of between 5 and 50 nm, nanofibres having a range of lengths from 10 - 500 nm, nanotubes having diameters from 5 - 100 nm and lengths from 20 nm - 1 µm, or other nanomaterial having photocatalytic characteristics, are chemically functionalised with the wall of the surface of the micro- or nanocapsules, in the interior thereof comprising the diffusing agent in an available volume of 10⁻²⁵ - 10⁻⁵ mL, in particular 10⁻¹⁵ - 10⁻¹⁰ mL. Optionally, the nanoparticles of titanium dioxide, or the derivatives thereof, may be on the internal or external part of the wall of the capsule, or on both, or in the very microstructure of the wall of the micro- or nanocapsules.

In this manner, nanocapsules are all the capsules comprising a diameter from 0.1-1 µm. The microcapsules are all the capsules comprising a diameter from 1 - 500 µm.

A nanomaterial is defined as a nanoparticle, nanotube or nanofibre comprising in the composition thereof aggregates of unitary cells of one or more photocatalytic compounds having a size smaller than 1 micrometre.

The photocatalytic compounds are semiconductors which absorb energy and give rise to oxidation-reduction reactions responsible for the degradation or rupture of the microcapsule or nanocapsule and the subsequent release of an active agent.

An active agent is a compound located in the nucleus of the capsule, in the liquid, solid or gaseous state and the release whereof is realised by degradation or rupture of the microcapsule or nanocapsule.

The solar radiation on illuminating the semiconductive surface of the photocatalytic material will initiate mechanisms of oxidation-reduction which will degrade or open the pores of the polymeric nano- or microcapsules containing the diffusing agent, promoting the controlled release thereof and enhancing the desired effect. To aid in the chemical functionalization of nanomaterials of titanium dioxide on the surface of the polymeric microcapsules or nanocapsules there may be used external chemical compounds having chemical affinity for both. By virtue of the affinity of TiO₂ with reactive hydroxyl groups (-OH), compounds having such a reactive group in the structure thereof, in particular polyethers such as polyethylene glycol, polyethylene oxide and polypropylene oxide may be used. Polyols capable of increasing the density of hydrogen bonds, and thereby promoting the bond between the microcapsules or nanocapsules and the nanomaterials based on titanium dioxide may also be used. Compounds intrinsic to the microcapsules or nanocapsules, in particular amines (-NH₂), may also be used. This group of compounds is one of the constituent monomers of the wall of microcapsules obtained by interfacial polymerisation, and when used in excess during the synthesis renders the unused -NH₂ groups for the formation of the wall to become available for binding to the TiO₂ structure allowing a homogenous coating of the microcapsules with the nanomaterials, such as, for example, nanoparticles of TiO₂.

At pH levels higher than the isoelectric point thereof (pH = 6), the nanoparticles with photocatalytic compounds, in particular of titanium dioxide, present a negative charge by virtue of the accumulation of electrons on the surface thereof. The incorporation during the synthesis of cationic compounds into the wall of the microcapsules allows the chemical bonding between the nanoparticles and the microcapsules, by virtue of the electronic attraction between the two compounds. Examples of cationic (positively charged) compounds which may be used are the quaternary ammonium salts, such as tetramethylammonium hydroxide, cetrimonium chloride, cetrimonium bromide, and benzalkonium chloride.

This technology is characterised in that it may be applied on untreated static substrates, simply by attaching onto the substrate through spraying, or deposition with or without the use of cationic or anionic surfactants, dependent on the electrostatic attraction between the surfaces of the system of functionalised microcapsules with the encapsulated photocatalytic nanomaterials and certain agents.

For the binding of the microcapsules or nanocapsules to the different substrates acrylic compounds such as acrylic acid, ethyl acrylate, methyl acrylate, hydroxyethyl acrylate and hydroxyethyl methacrylate may be used. Synthetic latexes such as styrene-butadiene may also be used, as may cellulose derivatives. Polyvinyl acetate is also one of the polymers most used for binding to wood substrates. The use of surfactants is employed principally with quaternary cationic ammonium salts, such as tetramethylammonium hydroxide, cetrimonium chloride, cetrimonium bromide, and benzalkonium chloride.

In one embodiment, the substrate may consist of clothing, tent, mosquito net, curtains, awnings, glazed surfaces, varnished or painted surfaces or metal, ceramic or polymeric panels, wood, or any other substrate or structure with direct solar exposure or equivalent ultraviolet radiation. In order for the diffusing agent to be released a mechanical initiation of the microcapsule or the nanocapsule wall is not required, by virtue of the fact that this activation will solely and exclusively be realised by oxidation/reduction mechanisms associated with the intrinsic process of the photocatalysis of the nanomaterials functionalised on the exterior wall of the micro- or nanocapsule.

In one embodiment, the synthesis of the photocatalytic nanomaterials is accomplished by a hydrothermal chemical process in an autoclave commencing from a particular precursor. The microcapsules or nanocapsules are subsequently synthesised by a process of interfacial polymerization or by the phase inversion technique, wherein the photocatalytic nanomaterials and the active agent are added.

The present invention relates to capsules for transporting an active agent having photocatalytic properties, having an external diameter from 0.05 - 500 µm, preferably 1 - 500 µm,
wherein the capsule is formed by a wall and a nucleus to lodge the diffusing agent, wherein the capsule wall comprises a polymeric film selected from the list constituted by poly(methyl methacrylate), melamine-formaldehyde, polyurethane, polysulfone, cellulose acetate, and mixtures thereof;
wherein the polymeric film comprises nanomaterials, such as nanoparticles, nanotubes or nanofibres chemically functionalised with a photocatalytic compound selected from a list: TiO₂, WO₃, WS₂, Nb₂O₅, MoO, MoS₂, V₂O₅, MgF₂, Cu₂O, NaBiO₃, NaTaO₃, SiO₂, RuO₂, BiVO₄, Bi₂WO₆, Bi₁₂TiO_{2C}, NiO-K₄NB₆O₁₇, SrTiO₃, Sr₂NbO₇, Sr₂TaO₇, BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe, and mixtures thereof;
the active agent/diffusing agent being located in the nucleus in liquid, solid or gaseous state wherein the distribution of the photocatalytic nanomaterials upon the surface of the capsule is 0.1 - 5% w/v of total capsule;
wherein the photocatalytic nanomaterials are chemically functionalised upon the exterior surface of the wall of the capsule.By virtue of the great mechanical strength thereof these capsules are especially appropriate for transporting the active agent/diffusing agent in the solid or liquid state.

In one embodiment, the wall of the capsule is from 55 - 80% w/v_{capsule wall} of a polymeric film and 20 - 45% w/v_{capsule wall} of photocatalytic nanomaterials.

In one embodiment, the photocatalytic nanomaterials are dispersed, chemically functionalised, upon the exterior surface of the wall of the capsule, or upon the inner surface of the wall of the capsule or bound to the wall of the capsule.

In one embodiment, the polymeric film may be selected from the list consisting of: polysulfone, poly(methyl methacrylate), polyurethane, or mixtures thereof.

In one embodiment, the capsules comprise a polymeric film of poly(methyl methacrylate) and dispersed nanomaterials comprising a photocatalytic material selected from a list: TiO₂, WO₃, SrTiO₃, ZnO, or mixtures thereof.

In one embodiment, the capsules comprise a polymeric film of polyurethane and dispersed nanomaterials comprising a photocatalytic material selected from a list: TiO₂, WO₃, SrTiO₃, ZnO, or mixtures thereof.

In one embodiment, the capsules comprise a polymeric film of polysulfone and dispersed nanomaterials comprising a photocatalytic material selected from a list: TiO₂, WO₃, SrTiO₃, ZnO, or mixtures thereof.

In one embodiment, the diameter of the capsule ranges from 0.1 - 500 µm.

In one embodiment, the nanomaterials in the form of nanoparticles have a diameter of between 5 and 50 nm; the nanomaterials in the form of nanofibres have a range of lengths from 10 - 500 nm; the nanomaterials in the form of nanotubes have diameters from 5 - 100 nm and lengths from 20 nm - 1 µm.

In one embodiment, the thickness of the wall of the capsule ranges from 0.05 - 25 µm; in particular 0.2 - 10 µm.

In one embodiment, the wall of the capsule is formed of a plurality of layers.

In one embodiment, the volume of the active agent ranges from 10⁻²⁵ - 10⁻⁵ mL, in particular 10⁻¹⁵ - 10⁻¹⁰ mL.

In one -embodiment, the active agent may be an insect repellent, an insecticide, a therapeutic agent, a radiotherapy agent, a deodorising agent, a natural essence, a fragrance, a moisturising agent, a component of a varnish or paint, or an agrochemical.

In one embodiment, the capsules may further comprise at least a surfactant, an emulsifier, a binder, or mixtures thereof.

In one embodiment, the surfactant is selected from the following list: tetramethylammonium hydroxide, cetrimonium chloride, cetrimonium bromide and benzalkonium chloride.

In one embodiment, the active agent may be hydrophobic.

In one embodiment, the capsules may be obtainable by interfacial polymerization.

The present invention also relates to articles comprising at least one afore described capsule, in particular these articles may be textiles, fibres, glass, wood, metal, tents, mosquito nets, resins, paints, curtains, detergents, softeners, creams, foams, or colloidal suspensions.

The present invention furthermore relates to a process for obtaining the afore described capsules and which may comprise the following steps:
preparation of an organic solution comprising 5 - 30% (w/v) of a reactive compound selected from the following list: 2,4-toluene diisocyanate, 2,4-diphenylmethane diisocyanate, 1,6-hexamethylene diisocyanate;
preparation of an organic solution comprising 70 - 95% (w/v) of a hydrophobic active agent; stirring the organic solution, in particular for 1 - 2 min;
preparation of an aqueous solution comprising an emulsifier, a colloidal agent, or mixtures thereof, in a particular embodiment the emulsifier being gum arabic (15 - 20% w/v), Tween 20 (1 - 3% v/v) or mixtures thereof and wherein the colloidal agent is poly(vinyl acid) (1 - 3% w/v);
formation of an oil/water emulsion with the foregoing solutions, preferably under mechanical stirring at 400 - 1200 rpm for 3 - 8 min;
addition to the emulsion of a hydrophilic monomer selected from the following list: ethylenediamine, diethylenetriamine, hexamethylenediamine, p-phenylenediamine, 1,4-butanediol, 1,6-hexanediol, ethylene glycol or polyethylene glycol in a range of concentrations comprised between 0.2 and 1 mol/dm³;
stirring the emulsion, preferably at 400 - 800 rpm for 10 - 60 min, preferably for 40 min;
collection of the nano- or microcapsules obtained, in particular by centrifugation or filtration at ambient temperature,
dispersal of the nano- or microcapsules collected in aqueous solutions comprising 10 - 20% v/v of amines, polyols, polyethers, or mixtures thereof;
addition to the suspension of nano- or microcapsules obtained of a nanomaterial, such as a nanoparticle, nanotube or nanofibre comprising a photocatalytic material, wherein the nanomaterial is selected from the following list: TiO₂, WO₃, WS₂, Nb₂O₅, MoO, MoS₂, V₂O₅, MgF₂, Cu₂O, NaBiO₃, NaTaO₃, SiO₂, RuO₂, BiVO₄, Bi₂WO₆, Bi₁₂TiO_{2C}, NiO-K₄NB₆O₁₇, SrTiO₃, Sr₂NbO₇, Sr₂TaO₇, BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe, or mixtures thereof.

In one form of embodiment, the ratio of hydrophilic to hydrophobic monomer concentrations is 3:1, 4:1, or 5:1.

In one form of embodiment, the percentage of gum arabic used is 15 - 20% w/v.

In one form of embodiment, the percentage of Tween 20 used is 1 - 2% v/v.

In one form of embodiment, the percentage of poly(vinyl acid) used is 1 - 3% w/v.

In one form of embodiment, the capsules comprise a hydrophilic active agent encapsulated therein.

In one form of embodiment, the capsules are obtained by the phase inversion technique.

In one form of embodiment, the process of obtaining the afore described capsules comprises the following steps:
preparation of an organic solution comprising 10 - 20% w/v of a polymer selected from the following list: polysulfone, cellulose acetate, poly(methyl acrylate), and polyacrylonitrile;
preparation of an organic solution comprising 80 - 90% v/v of a volatile solvent selected from the following list: dichloromethane, N,N-dimethylformamide, acetone, and chloroform;
stirring the organic solution, in particular for 23 h;
preparation of an aqueous solution comprising hydrophilic diffusing agent;
formation of a water/oil emulsion with the previous solutions, preferably under mechanical stirring at 400 - 1200 rpm for 2 - 8 h;
immersion of the emulsion in a bath containing a non-solvent, in particular water;
collection of the nano- or microcapsules obtained, in particular by centrifugation or filtration at ambient temperature;
dispersal of the nano- or microcapsules collected in aqueous solutions comprising 10 - 20% v/v of amines, polyols, polyethers, or mixtures thereof;
addition to the suspension of nano- or microcapsules of a nanomaterial, such as a nanoparticle, nanotube or nanofibre comprising a photocatalytic material wherein the nanomaterial is selected from the following list: TiO₂, WO₃, WS₂, Nb₂O₅, MoO, MoS₂, V₂O₅, MgF₂, Cu₂O, NaBiO₃, NaTaO₃, SiO₂, RuO₂, BiVO₄, Bi₂WO₆, Bi₁₂TiO₂₀, NiO-K₄NB₆O₁₇, SrTiO₃, Sr₂NbO₇, Sr₂TaO₇, BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe, or mixtures thereof.

Process according to the preceding claim wherein the step of this version of the nano- or microcapsules is realised by dispersion in an aqueous solution comprising one or more surfactants.

In one form of embodiment, the step of addition of the photocatalytic nanomaterial is realised at a basic pH, in particular from 9 - 11.

In one form of embodiment, the polyol is selected from the following list: 1,4-butanediol, ethylene glycol, 1,6-butanediol, or mixtures thereof.

In one form of embodiment, the polyether is selected from the following list: polyethylene glycol, polyethylene oxide, polypropylene oxide, or mixtures thereof.

### Process for the preparation of the microcapsules or nanocapsules:

In general terms, the process is initiated through polymerization or precipitation reactions leading to the formation of microcapsules or nanocapsules which may be based on polyurethane inter alia other polymers such as parylene, poly(p-xylylene), poly(acid lactic), poly(ε-caprolactone), polyoxyethylenated derivatives, phthalocyanine, polysulfone, polystyrene, cellulose acetate, acrylic polymers, collagen, or chitosan encapsulating the diffusing agent which it is intended to be released, which may be in the liquid, solid or gaseous state. Subsequently, the photocatalytic nanomaterials, that is to say nanoparticles, nanotubes, or nanofibres, based on TiO₂, or another type of nanomaterials having demonstrated photocatalytic activity, such as based on WO₃, WS₂, Nb₂O₅, MoO, MoS₂, V₂O₅, MgF₂, Cu₂O, NaBiO₃, NaTaO₃, SiO₂, RuO₂, BiVO₄, Bi₂WO₆, Bi₁₂TiO₂₀, NiO-K₄NB₆O₁₇, SrTiO₃, Sr₂NbO₇, Sr₂TaO₇, BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, or CdSe, synthesised by a hydrothermal synthesis process in autoclave, are added to the solution of the microcapsules or nanocapsules, under the effect of mechanical homogenisation. The process having finished, microcapsules or nanocapsules are obtained which, by solar activation, the oxidation/reduction (redox) mechanisms initiated by the photocatalytic nanomaterials lead to the degradation or rupture of the wall of the microcapsule or nanocapsule, promoting the diffusion of the specific agent which was encapsulated.

More specifically, for the microencapsulation of hydrophobic diffusing agents, the interfacial polymerization technique is used based on the interfacial reaction between different monomers solubilised in different phases. The first stage of the process of microencapsulation is the emulsification, wherein one of the monomers containing the diffusing agent is solubilised in an aqueous disperse phase. In one embodiment, prior to the step of emulsion, an organic solution containing 0.1 to 5 mL of diffusing agent and 0.25 to 8 mL of organic monomer is prepared, under vortex stirring for 1 to 2 min. Various organic monomers are used to promote the formation of the wall of the microcapsule or nanocapsule, depending on the type of desired polymer. In the case of polyurethane, the monomers 2,4-toluene diisocyanate, 2,4-diphenylmethane diisocyanate, and 1,6-hexamethylene diisocyanate are used. The next step of the process is the formation of an oil-in-water (O/W) emulsion for the use of oils as diffusing agents. Under mechanical stirring (400 - 1200 rpm), the organic solution is dispersed in the aqueous phase containing an emulsifier (15 - 20% gum arabic and 1- 2% Tween 20), or a colloidal agent (1 - 3% polyvinyl alcohol). To ensure the stability thereof the emulsion is stirred for a period of time of 3 to 8 min. The size of the final microcapsules or nanocapsules is directly related to the size of the droplets of the emulsion resulting from the rupture of the oil phase by the action of surface tension and intermolecular collisions caused by the mechanical agitation.

In the final stage of the process, an aqueous solution containing the hydrophilic monomer, in a range of concentration between 0.2 and 1 mol/dm³ is added. For polyurethane coatings, the hydrophilic monomers used are polyols, such as 1,4-butanediol, 1,6-hexanediol, ethylene glycol, or polyethylene glycol. The addition of these monomers to the emulsion initiates the polymerisation reactions between the organic monomer and the hydrophilic monomer resulting in a polymeric film at the interface of the already emulsified oil droplets, giving rise to the wall of the microcapsules or nanocapsules. The suspension of microcapsules or nanocapsules formed is maintained under stirring for a maximum time of 40 minutes for the maturation and stabilization of the polymeric coating around the microcapsule. The speed of stirring during the process ranges from 400 to 800 rpm. The final microcapsules or nanocapsules are furthermore subjected to a washing process with cyclohexane or water for the removal of the excess solvents. In one embodiment, for the incorporation of the nanoparticles, nanotubes or nanofibres, based on TiO₂ upon the surface of the microcapsules or nanocapsules during the process of synthesis, monomers having chemical affinity for such materials, in particular amines, are used. Using an excess concentration of the hydrophilic monomer in relation to the hydrophobic monomer, the OH reactive groups will be chemically available for the reaction of polymerisation with the organic monomer and furthermore for chemical bonding to the titanium dioxide. For this procedure, ratios of concentration of hydrophilic and hydrophobic monomers used are 3:1, 4:1, or 5:1. For the chemical adsorption of the TiO₂ nanoparticles to be effective it is necessary that the pH of the suspension of the microcapsules is alkaline, having values from 9 - 11.

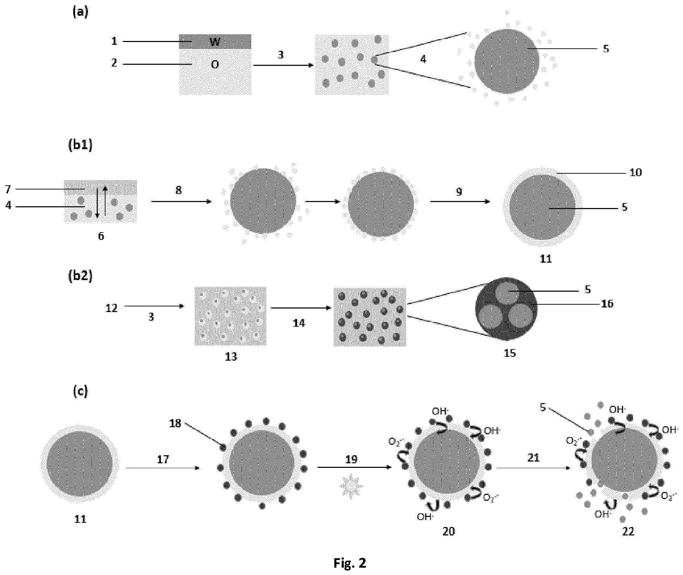

In one embodiment, for the microencapsulation of hydrophilic active compounds, the invention employs the phase inversion technique. The precipitation of the microcapsules or microspheres may be induced by a process of immersion or by evaporation of the solvent. For both processes the first stage consists in preparing a primary water-in-oil (W/O) emulsion. An aqueous solution containing the active agent is added to a polymeric solution and emulsified through mechanical stirring for a period of time of between 2 and 8 hours, forming the W/O emulsion (Figure 2). Prior to the stage of emulsification, the polymeric solution is prepared by dissolving the polymer in a suitable solvent under magnetic stirring for 2 - 3 h. Different polymers may be used, such as polysulfone, cellulose acetate, poly(methyl acrylate) and polyacrylonitrile. The concentration of the polymer in solution must be 10 to 20% (w/v). The used solvent must be capable of solubilising the polymer, have low solubility in water, high volatility and low toxicity. Among the most common solvents there may be highlighted dichloromethane, N,N-dimethylformamide, acetone and chloroform. The precipitation of the microcapsules may take place through the immersion of the primary emulsion in a bath containing a non-solvent, represented in the diagram of Figure 2 by b1, or by the technique of solvent evaporation, represented by b2. In the first case, the final microcapsules are obtained by dispersing the W/O primary emulsion in the form of microdroplets in a water bath. The polymeric coating of the active agent occurs by a rapid process of gelatinisation (5 - 10 s), based on processes of diffusion between the solvent and the non-solvent, leading to the separation and the precipitation of the polymer around the active agent.

In addition to the technique of immersion, the formation of the microcapsules may be induced by evaporation of the solvent of the polymeric solution (Figure 2 b2). As in the foregoing process, the first stage of the microencapsulation consists in the homogeneous dispersion of the active agent in the solution of polymer and of the volatile solvent, yielding a primary water-in-oil (W/O) emulsion. This emulsion is then added to an aqueous solution containing one or more emulsifiers to form a double water-in-oil-in-water (W/O/W) emulsion. Amongst the most used emulsifiers highlighted Tween 20 (1 - 2%) or poly(vinyl acid) (1 - 3%) may be selected. The double W/O/W emulsion is generated mechanically in vigorous manner until the evaporation of the volatile solvent is complete, leading to the precipitation of the polymer and formation of the capsules.

This technique may be used for the microencapsulation of solid active compounds, having as principal difference the time of stirring the solution containing the polymer, the solvent and the active agent. In this case, the mixture must be stirred for between 12 and 24 hours to ensure that the polymeric coating of the solid is homogeneous.

For both processes the microcapsules may be harvested by centrifugation or filtration and dried at ambient temperature.

Subsequent to the synthesis and washing of the microcapsules obtained by the different techniques the coating with the nanomaterials based on photocatalytic titanium dioxide is proceeded to. In the case of nanoparticles of TiO₂, these are dispersed in aqueous solution having a pH exceeding 9, using ultrasound for 30 min following this period of time, the photocatalytic nanoparticles are added to the suspension of microcapsules under mechanical stirring using a shaft of the propeller type at a speed of 400 rpm. The mixture continues to be stirred for 30 min and is then collected. The resulting microcapsules containing nanoparticles of titanium dioxide adsorbed onto the surface thereof remain in aqueous dispersion or are filtered and dried in the oven at 40 °C.

The process of coating the micro- or nanocapsules with nanomaterials based on titanium dioxide, such as the nanoparticles, may also be achieved by using external compounds having affinity for the nanoparticles, in particular compounds having reactive -OH groups. Examples of such compounds are polyethylene glycol, polyethylene oxide and polypropylene oxide. Chain extenders such as 1,4-butanediol, ethylene glycol or 1,6-hexanediol may also be used to increase the density of hydrogen bonds on the wall of micro- or nanocapsules. For this type of process, the nanomaterials of titanium dioxide are solubilised in the stated solvents and incorporated into the micro- or nanocapsules subsequent to the production and washing thereof.

### Brief description of the figures

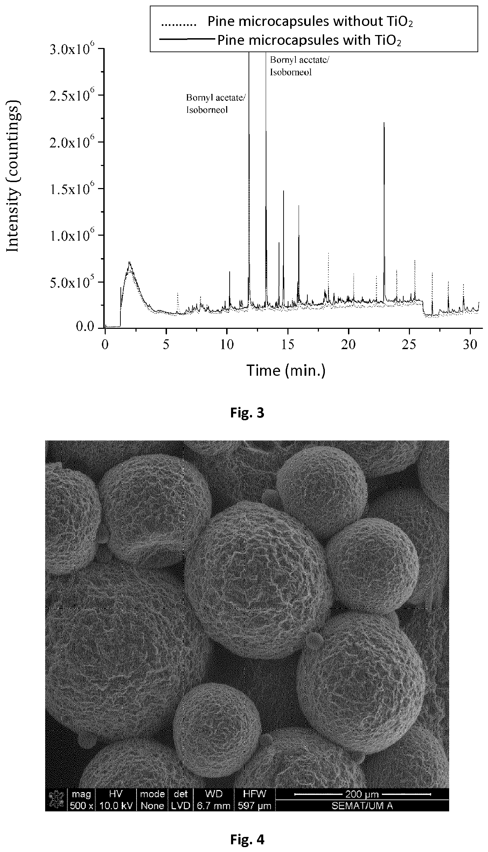

For easier understanding of the solution the figures are attached in an annex, representing preferred embodiments of the solution here and divulged which, nevertheless, do not have the intention of limiting the object of the present application.

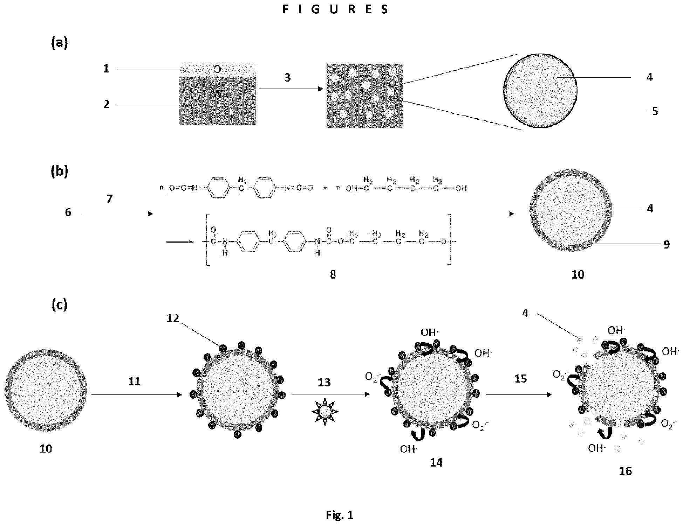

**Figure 1****:** Diagram representing the production of the system for controlled release of hydrophobic diffusing agents by solar activation in samples of microcapsules having a polyurethane film chemically functionalised with photocatalytic nanomaterials, wherein:
   **(a)** EMULSION PREPARATION
      1- Organic phase: hydrophobic monomer (e.g. 4,4-diphenyl diisocyanate) + active agent
      2- Aqueous phase: aqueous solution with emulsifier (e.g. PVA, Tween 20)
      3- Emulsification: 3 to 5 min
      4- Droplet of the active agent
      5- Formation of the polymeric premembrane
   **(b)** PRECIPITATION OF THE POLYMER AND MICROENCAPSULATION
      6- Addition of the hydrophilic monomer (e.g. 1,4-butanediol) to the emulsion
      7- Mechanical stirring
      8- Condensation reactions between the reactive monomers
      9- Polymeric wall
      10- Polymeric microcapsule containing the active agent
   **(c)** CONTROLLED RELEASE OF THE ACTIVE AGENT
      11- Coating of the microcapsules with nanoparticles of TiO₂
      12- Nanoparticles of TiO₂
      13- UV irradiation
      14- Oxidation-reduction reactions on the surface of the wall of the microcapsule functionalised with TiO₂
      15- Rupture of the polymeric wall of the microcapsule
      16- Release of the active agent from the interior of the microcapsule
**Figure 2****:** Diagram representing the production of the system for controlled release of hydrophilic diffusing agents by solar activation in samples of microcapsules having a poly(methyl methacrylate) or polysulfone film chemically functionalised with photocatalytic nanomaterials, wherein:
   **(a)** PREPARATION OF THE PRIMARY EMULSION
      1- Aqueous phase: aqueous solution of the hydrophilic active agent
      2- Organic phase: polymeric solution (e.g. poly(methyl methacrylate) or polysulfone) containing a volatile solvent
      3- Emulsification
      4- Primary water-in-oil emulsion
      5- Droplet of the hydrophilic active agent
   **(b1**) PRECIPITATION OF THE POLYMER BY IMMERSION AND MICROENCAPSULATION
      6- Immersion of the primary emulsion in a precipitation bath containing a non-solvent
      7- Solution of a non-solvent (e.g. water)
      8- Diffusion of the solvent into the bath and of the non-solvent into the polymeric solution
      9- Precipitation of the polymer
      10- Polymeric wall
      11- Polymeric microcapsule containing the active agent
   **(b2)** PRECIPITATION OF THE POLYMER BY EVAPORATION OF THE SOLVENT AND MICROENCAPSULATION
      12- Dispersion of the primary emulsion in an aqueous solution containing one or more emulsifiers
      13- Double water-in-oil-in-water emulsion
      14- Evaporation of the solvent
      15- Precipitation of the polymer
      16- Polymeric matrix
   **(c)** CONTROLLED RELEASE OF THE ACTIVE AGENT
      17- Coating of the microcapsules with TiO₂ nanoparticles
      18- Nanoparticles of TiO₂
      19- UV irradiation
      20- Oxidation-reduction reactions on the wall of the surface of the microcapsule functionalised with TiO₂
      21- Rupture of the polymeric wall of the microcapsule
      22- Release of the active agent from the interior of the microcapsule
**Figure 3****:** Graph illustrating the evaluation, by gas chromatography coupled with mass spectrometry, of the controlled release from samples of microcapsules loaded with a pine fragrance (for example), with and without TiO₂ nanoparticles chemically functionalised on the exterior wall of the microcapsule. When the microcapsules are functionalised with the photocatalytic nanoparticles the release of molecules of the essence of pine (bornyl acetate/isoborneol) which are absorbed into the PDMS fibre is much greater.
**Figure 4****:** Example micrograph of photocatalytic microcapsules of the present invention loaded with a diffusing agent.

### Detailed description

An example of photocatalytic nanomaterials are the nanoparticles based on TiO₂. These materials are synthesised using a hydrothermal sol-gel process in an autoclave. A colloidal solution is prepared with water and 2-propanol (10:1). As an example, 125 µL of 2-propanol and 1125 µL of water are mixed at ambient temperature and under vortex stirring in a homogeniser at a pH of 2.40 (adjusted with a solution of 0.1 M HCI). Optionally, the nanoparticles based on TiO₂ may be prepared with triethylamine for them to be doped with nitrogen for the purpose of increasing the semiconductor band-gap energy and the efficiency of absorption of solar light. Under strong magnetic stirring at 400 - 600 rpm and at ambient temperature 1000 µL of titanium isopropoxide (precursor source of atoms of titanium) are added to a volume of 1250 µL of a colloidal solution. In the case of synthesis of doped particles 3000 µL of triethylamine are added to the resulting white suspension. The amine is responsible for doping of the TiO₂ with nitrogen. In order for the doping of the particles of TiO₂ with nitrogen to occur, it is necessary to leave the reaction under magnetic stirring for 2 days. Following this period, 10 mL of water and 10 mL of 2-propanol are added to the suspension and the mixture is placed in an autoclave at 200 °C for 2 hours. Following cooling to ambient temperature, the washing of the particles is proceeded too. For this purpose, an organic solvent (2-propanol) is used to permit the precipitation of the particles subsequent to centrifugation. The process of washing is repeated several times in order to ensure that all the unreacted solvents are eliminated. The particles collected are dried in an oven at 80 °C for 8 h. Optionally, in order to reduce the size of the crystallites of nanoparticles to between 5 and 50 nm, the particles are placed in an oven to carry out the heat treatment at 635 °C and ensure the formation of the crystalline allotropic phases of the material, in this case anatase, preferably, and rutile, which demonstrate having catalytic photoactivity. Following all this process, the characterisation of the material is proceeded to: assessment of the photocatalytic activity in a photoreactor in the presence of a pollutant simulator; X-ray diffraction characterization experiment to determine the crystalline phases which have developed (anatase, rutile); dynamic light scattering characterization experiment to assess the size and size distribution of the nanoparticles; evaluation of the morphology of the particles using scanning electron microscopy.

An example of polymeric microcapsules obtained by interfacial polymerisation are the microcapsules having a polyurethane coating. In a first phase of the process, the organic solution is prepared by mixing 5 mL of active diffusing agent and 5 mL of organic monomer (4,4'-diphenylmethane diisocyanate) in an organic solvent (dichloromethane) under vortex stirring with a homogeniser, in particular for 2 min. In a second phase, the organic solution previously prepared is added dropwise to an aqueous solution of 2% PVA (polyvinyl alcohol) under mechanical stirring with a cowles-type rod at a speed of 1000 rpm. This polymer is used as emulsifying agent permitting the dispersion of the oil droplets of the organic solution in the aqueous phase. The formed emulsion is allowed to stir for 3 min.

Following this stage the stirring speed is reduced to 600 rpm and an aqueous solution of 1,4-butanediol is added, in particular at a concentration 0.32 mol/dm³ and a rate of 0.6 mL/min. The addition of the hydrophilic monomer initiates the reactions of polymerisation between the organic monomer and the hydrophilic monomer, resulting in a polymeric film of polyurethane at the interface of the already-emulsified droplets of oil, giving rise to the microcapsules wall. The addition being complete, the solution is allowed to be stirred for further 30 min to ensure that the process of polymerisation is complete in its entirety. This process is described in Figure 1.

In order to remove excesses of solvents it is necessary to proceed to washing the capsules with water and cyclohexane. The process is carried out by vacuum filtration using a polycarbonate membrane of 2 µm porosity. The microcapsules are collected and dispersed again in water.

An example of polymeric microcapsules obtained by the technique of phase inversion are the microcapsules of polysulfone containing in the interior thereof solid diffusing agents having hydrophilic properties. In a first phase of the process the polymeric solution constituting the wall of the final capsules is prepared by dissolving 1.5 g of polysulfone in 10 mL of N,N-dimethylformamide under magnetic stirring for 2h. Following the complete dissolution 0.5 g of the solid diffusing agent is added to the polymer solution. The suspension is allowed to be stirred magnetically for a period of time never less than 12 hours to ensure that the polymeric coating of the solid is homogeneous. Using a compressed air pistol, the polymeric suspension containing the diffusing agent is dispersed in the form of microdroplets into a water bath (200 mL) at ambient temperature. The process of precipitation is immediate and the microcapsules formed are collected by centrifugation or filtration and dried at ambient temperature. In order to remove excesses of solvents it is necessary to proceed to the washing of the capsules with water. The process is conducted by vacuum filtration using a porous polycarbonate membrane of 2 µm porosity. The size, distribution, morphology of the microcapsules are directly related to parameters such as the quantity of active agent, concentration of the emulsifier, concentration of the polymer, stirring speed, temperature and pressure.

Subsequent to the synthesis and washing of the microcapsules obtained by the different techniques the chemical functionalisation thereof with the nanomaterials based on photocatalytic titanium dioxide is proceeded too. In the case of nanoparticles of TiO₂, these are dispersed in aqueous solution having a pH exceeding 9, using ultrasound for 30 min. Following this period of time, the photocatalytic nanoparticles are added to a suspension of microcapsules under mechanical stirring, using a shaft of the propeller type at a speed of 400 rpm. The mixture is left to be stirred for 30 min and then collected. The resulting microcapsules containing chemically functionalised nanoparticles of titanium dioxide upon the surface thereof remain in aqueous dispersion or are filtered and dried in the oven at 40 °C.

To evaluate the success of the microencapsulation of the diffusing agent the analytical techniques of thermogravimetry (TGA) and Fourier transform infrared spectroscopy (FTIR) are used.

The pure diffusing agent, the polymeric wall, and the microcapsules previously dried at 40 °C for 6 h are evaluated in the FTIR analysis. The KBr powder (spectroscopic grade) is mixed in a mortar together with the pure diffusing agent and the dry polymeric wall or dry microcapsules (1%). The resulting powder is placed in a mould of 1 cm diameter and taken to a hydraulic press to form the translucent pellet used for the analysis. To prepare the samples the microcapsules are crushed and washed several times with water and ethanol. The chemical structure of the diffusing agent, polymeric wall and resulting microcapsules is characterised by FTIR in a range of wavelengths ranging from 400 cm⁻¹ to 4000 cm⁻¹. In a first analysis the spectrum obtained to determine the chemical bonds characteristic of the diffusing agent are evaluated. The presence of the characteristic absorption bands in the spectrum indexed to the diffusing agent allows for the conclusion that the diffusing agent is successfully encapsulated within the interior of the microcapsules. As an example, the analysis of the polyurethane wall permits it to be determined whether the process of polymerisation has been completed in its entirety by means of the presence of the absorption bands characteristic of the NH urethane bonds between 3300 and 3200 cm⁻¹, C=O bonds between 1730 and 1715 cm⁻¹, and N=C=O bonds between 1640 and 1600 cm⁻¹. For the thermogravimetric analyses, 10 - 20 mg of the previously dried microcapsules at 40 °C for 6h are placed into a Teflon or platinum crucible. The sample is heated at a temperature increasing from 60 to 600 °C under an argon atmosphere and at a rate of heating of 10 °C/min. The percentage of diffusing agent encapsulated within the resulting microcapsules is determined by the value of loss of mass associated with the temperature of ebullition or degradation of the diffusing agent; the loss of mass in relation to the degradation of the polymeric wall of the microcapsules occurs at temperatures exceeding 300 °C.

For the thermogravimetric analyses, 10 - 20 mg of microcapsules previously dried at 40 °C for 6h are placed into a Teflon or platinum crucible. The sample is heated at a rate of 10 °C/min from 60 to 600 °C under an argon atmosphere. The percentage of encapsulated diffusing agent within the resulting microcapsules is determined by the value of loss of mass associated with the temperature of ebullition or degradation of the diffusing agent; in the case of dodecane (example of diffusing agent) from 190 - 220 °C. The loss of mass in relation to the degradation of the polymeric wall of the microcapsules occurs at temperatures exceeding 300 °C.

For a quantitative analysis of the diffusing agent encapsulated within the interior of the microcapsule the technique of gas chromatography coupled with mass spectrometry is used. Analyses are performed in a chromatograph with equipped a column provided with an ion trap detector having an ionization energy of 70 eV. The method of solid phase microextraction (SPME), in the headspace mode, is used as extraction technique.

To quantify the output release of the diffusing agent it is necessary to analyse samples of polymeric microcapsules loaded with a diffusing agent, with or without titanium dioxide nanoparticles functionalized with the microcapsule adsorbed upon the surface of the wall, under UV irradiation (5 mW/cm²) and in the dark.

For the preparation of the samples to be analyzed by gas chromatography, the microcapsules are placed within a hermetically sealed vial for 2 h under UV irradiation and in the dark. Following this period of time, a polymeric fibre of PDMS (polydimethylsiloxane) having a length of 10 mm is injected into the interior of the vial without coming into direct contact with the microcapsules sample, but solely with the vapour phase, adsorbing the volatile analytes of the sample. Immediately following the extraction, the fibre is collected and injected into the gas chromatograph. The collected analytes are separated and detected by the equipment. The diffusing agent is identified through the analysis of the chromatograms and mass spectra obtained for each sample. The concentration thereof is determined through a linear regression obtained from the calibration curve relating the calculated peak area from the integration of the peaks from the chromatogram and the mass of the compound. The calibration curve is obtained by the injection of standards containing known masses of the diffusing agent.

Although in the detailed description of this example solely particular embodiments of the solution have been shown and described, a person skilled in the art will know how to introduce modifications and substitute some technical characteristics for others being equivalent, depending on the requirements of each situation, without diverging from the scope of protection defined by the appended claims.

The embodiments presented are combinable one with another. The following claims additionally define preferential embodiments.

## Claims

1. Capsules for transporting an active agent having photocatalytic properties, having an external diameter from 0.05-500 µm, wherein the capsule is formed by a wall and a nucleus to lodge the diffusing agent
wherein the capsule wall comprises a polymeric film selected from the list constituted by poly(methyl methacrylate), melamine-formaldehyde, polyurethane, polysulfone, cellulose acetate, and mixtures thereof,
wherein the polymeric film comprises upon the external surface nanomaterials, such as nanoparticles, nanotubes or nanofibres chemically functionalised with a photocatalytic compound selected from a list: TiO₂, WO₃, WS₂, Nb₂O₅, MoO, MoS₂, V₂O₅, MgF₂, Cu₂O, NaBiO₃, NaTaO₃, SiO₂, RuO₂, BiVO₄, Bi₂WO₆, Bi₁₂TiO_{20'} NiO-K₄NB₆O₁₇, SrTiO₃, Sr₂NbO₇, Sr₂TaO₇, BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe, and mixtures thereof,
and the nucleus comprises at least one active agent in liquid, solid or gaseous state wherein the distribution of the photocatalytic nanomaterials upon the surface of the capsule is 0.1 - 5% w/v of total capsule;
wherein the photocatalytic nanomaterials are chemically functionalised upon the exterior surface of the wall of the capsule.

2. Capsules according to the preceding claim, wherein the wall of the capsule is a distribution of polymeric film comprising from 55 - 80% w/v of total wall and of photocatalytic nanomaterials comprising from 20 - 45% w/v of total wall.

3. Capsules according to any one of the preceding claims, wherein the polymeric film is selected from the list constituted by: poly(methyl methacrylate), polysulfone, polyurethane, or mixtures thereof.

4. Capsules according to any one of the preceding claims, wherein the capsules comprise a polymeric film of poly(methyl methacrylate) and chemically functionalised nanomaterials upon the exterior surface thereof comprising a photocatalytic material selected from a list: TiO₂, WO₃, SrTiO₃, ZnO, or mixtures thereof.

5. Capsules according to any one of the preceding claims, wherein the nanomaterials in the form of nanoparticles have a diameter of between 5 and 50 nm.

6. Capsules according to any one of the preceding claims, wherein the nanomaterials in the form of nanofibres have lengths ranging from 10 - 500 nm.

7. Capsules according to any one of the preceding claims, wherein the nanomaterials in the form of nanotubes have diameters of 5 - 100 nm, and lengths from 20 nm - 1 µm.

8. Capsules according to any one of the preceding claims, wherein the thickness of the wall of the capsule ranges from 0.05 - 25 µm; in particular 0.2 -10 µm.

9. Capsules according to any one of the preceding claims, wherein the wall of the capsule is formed of a plurality of layers.

10. Capsules according to any one of the preceding claims, wherein the volume of the active agent ranges from 10⁻²⁵ - 10⁻⁵ mL, in particular 10⁻¹⁵ - 10⁻¹⁰ mL.

11. Capsules according to any one of the preceding claims, wherein the active agent is an insect repellent, an insecticide, a therapeutic agent, a radiotherapy agent, a deodorising agent, a natural essence, a fragrance, a moisturising agent, a component of a varnish or paint, or an agrochemical.

12. Capsules according to any one of the preceding claims, further comprising at least a surfactant, an emulsifier, a binder, or mixtures thereof; in particular wherein the surfactant is selected from the following list: tetramethylammonium hydroxide, cetrimonium chloride, cetrimonium bromide and benzalkonium chloride.

13. Articles comprising at least one capsule described in the preceding claims, in particular wherein the articles are textiles, fibres, glass, wood, metal, tents, mosquito nets, resins, paints, curtains, detergents, softeners, creams, foams or colloidal suspensions.

14. Procedure for obtaining the capsules for transporting an active agent having photocatalytic properties described in any one of claims 1 - 13, comprising the following steps:
preparation of an organic solution comprising 5 - 30% w/v of a reactive compound selected from the following list: 2,4-toluene diisocyanate, 2,4-diphenylmethane diisocyanate, 1,6-hexamethylene diisocyanate or 10 - 20% w/v of a polymer selected from the following list: polysulfone, poly(methyl acrylate), cellulose acetate, melamine-formaldehyde, polyurethane and mixtures thereof;
preparation of an organic solution comprising a volatile solvent selected from the following list: dichloromethane, N,N-dimethylformamide, acetone, and chloroform; 70 - 95% w/v of a hydrophobic active agent;
stirring the organic solution, in particular for 1 - 2 min;
preparation of an aqueous solution comprising an emulsifier, a colloidal agent, or mixtures thereof, in particular the emulsifier being gum arabic 15 - 20% w/v, Tween 20 1- 3% v/v or mixtures thereof and wherein the colloidal agent is poly(vinyl acid) 1 - 3% w/v;
addition of the active diffusing agent into the organic or aqueous solution;
formation of an oil/water emulsion with the foregoing solutions, preferably under mechanical stirring at 400 - 1200 rpm for 3 - 8 min;
for the encapsulation of hydrophobic active agents using the reactive monomers 2,4-toluene diisocyante, 2,4-diphenylmethane diisocyanate, 1,6-hexamethylene diisocyanate, add to the emulsion a hydrophilic monomer selected from the following list: ethylenediamine, diethylenetriamine, hexamethylenediamine, p-phenylenediamine, 1,4-butanediol, 1,6-hexanediol, ethylene glycol or polyethylene glycol in a range of concentrations comprised between 0.2 and 1 mol/dm³;
for the encapsulation of hydrophilic active agents using polysulphone, poly(methyl methacrylate), cellulose acetate add the emulsion to a precipitation bath followed by evaporation of the solvent;
stirring the emulsion, preferably at 400 - 800 rpm for 10 - 60 min, preferably for 40 min;
collection of the nano- or microcapsules obtained, in particular by centrifugation or filtration at ambient temperature,
dispersal of the nano- or microcapsules collected in aqueous solutions comprising 10 - 20% v/v of amines, polyols, polyethers, or mixtures thereof;
addition to the suspension of the obtained nano- or microcapsules a nanomaterial, such as a nanoparticle, nanotube or nanofibre comprising a photocatalytic material wherein the nanomaterial is selected from the following list: TiO₂, WO₃, WS₂, Nb₂O₅, MoO, MoS₂, V₂O₅, MgF₂, Cu₂O, NaBiO₃, NaTaO₃, SiO₂, RuO₂, BiVO₄, Bi₁₂TiO₂₀, NiO-K₄NB₆O₁₇, SrTiO₃, Sr₂NbO₇, Sr₂TaO₇, BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe, or mixtures thereof.

## Patentansprüche

1. Kapseln zum Transport eines Wirkstoffs mit photokatalytischen Eigenschaften mit einem äußeren Durchmesser von 0,05 500 µm, wobei die Kapsel aus einer Wand und einem Kern zur Aufnahme des Diffusionsmittels besteht
wobei die Kapselwand einen Polymerfilm umfasst, der aus einer Liste bestehend aus Poly(methylmethacrylat), Melamin-Formaldehyd, Polyurethan, Polysulfon, Celluloseacetat und Mischungen davon ausgewählt wird,
wobei der Polymerfilm auf der äußeren Oberfläche Nanomaterialien wie Nanopartikel, Nanoröhren oder Nanofasern umfasst, funktionalisiert mit einer photokatalytischen Verbindung, ausgewählt aus einer Liste von: TiO₂, WO₃, WS₂, Nb₂O₅, MoO, MoS₂, V₂O₅, MgF₂, Cu₂O, NaBiO₃, NaTaO₃, SiO₂, RuO₂, BiVO₄, Bi₂WO₆, Bi₁₂TiO₂₀, NiO-K₄NB₆O₁₇, SrTiO₃, Sr₂NbO₇, Sr₂TaO₇,BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe und Mischungen davon,
und der Kern mindestens einen Wirkstoff in flüssigem, festem oder gasförmigem Zustand enthält, wobei die Verteilung der photokatalytischen Nanomaterialien auf der Oberfläche der Kapsel 0,1 - 5 % w/v der gesamten Kapsel beträgt;
wobei die photokatalytischen Nanomaterialien auf der äußeren Oberfläche der Kapselwand chemisch funktionalisiert sind.

2. Kapseln nach dem vorangehenden Anspruch, wobei die Wand der Kapsel eine Verteilung des Polymerfilms mit 55 - 80% w/v der Gesamtwand sowie von photokatalytischen Nanomaterialien, die 20 - 45% w/v der Gesamtwand entsprechen, umfasst.

3. Kapseln nach einem der vorangehenden Ansprüche, wobei der Polymerfilm aus einer Liste ausgewählt wird, bestehend aus: Poly(methylmethacrylat), Polysulfon, Polyurethan oder Mischungen davon.

4. Kapseln nach einem der vorangehenden Ansprüche, wobei die Kapseln einen Polymerfilm aus Poly(methylmethacrylat) und chemisch funktionalisierten Nanomaterialien auf der äußeren Oberfläche umfassen, die ein photokatalytisches Material enthalten, ausgewählt aus einer Liste, bestehend aus: TiO₂, WO₃, SrTiO₃, ZnO oder Mischungen davon.

5. Kapseln nach einem der vorangehenden Ansprüche, wobei die Nanomaterialien in Form von Nanopartikeln einen Durchmesser zwischen 5 und 50 nm aufweisen.

6. Kapseln nach einem der vorangehenden Ansprüche, wobei die Nanomaterialien in Form von Nanofasern eine Länge von 10 - 500 nm aufweisen.

7. Kapseln nach einem der vorangehenden Ansprüche, wobei die Nanomaterialien in Form von Nanoröhren einen Durchmesser von 5 - 100 nm und eine Länge von 20 nm - 1 µm aufweisen.

8. Kapseln nach einem der vorangehenden Ansprüche, wobei die Stärke der Kapselwand zwischen 0,05 - 25 µm, insbesondere 0,2 - 10 µm, liegt.

9. Kapseln nach einem der vorangehenden Ansprüche, wobei die Kapselwand aus mehreren Schichten gebildet wird.

10. Kapseln nach einem der vorangehenden Ansprüche, wobei das Wirkstoffvolumen zwischen 10⁻²⁵ - 10⁻⁵ ml, insbesondere zwischen 10⁻¹⁵ - 10⁻¹⁰ ml, liegt.

11. Kapseln nach einem der vorangehenden Ansprüche, wobei der Wirkstoff ein Repellent für Insekten, ein Insektizid, ein therapeutischer Wirkstoff, ein Wirkstoff zur Strahlentherapie, ein desodorierender Wirkstoff, eine natürliche Essenz, ein Duftstoff, ein feuchtigkeitsspendendes Mittel, ein Bestandteil eines Lacks oder einer Farbe oder eine Agrochemikalie ist.

12. Kapseln nach einem der vorangehenden Ansprüche, die ferner mindestens ein Tensid, einen Emulgator, ein Bindemittel oder Mischungen davon umfassen; wobei das Tensid insbesondere aus folgender Liste ausgewählt wird: Tetramethylammoniumhydroxid, Cetrimoniumchlorid, Cetrimoniumbromid und Benzalkoniumchlorid.

13. Artikel, die mindestens eine der in den vorangehenden Ansprüche beschriebene Kapsel enthalten, wobei es sich bei den Artikeln insbesondere um Textilien, Fasern, Glas, Holz, Metall, Zelte, Moskitonetze, Harze, Farben, Vorhänge, Waschmittel, Weichmacher, Cremes, Schäume oder kolloidale Suspensionen handelt.

14. Verfahren zum Erhalt der Kapseln für den Transport eines Wirkstoffs mit photokatalytischen Eigenschaften nach einem der Ansprüche 1 - 13, umfassend die folgenden Schritte:
Herstellung einer organischen Lösung, die 5 - 30 % w/v einer reaktiven Verbindung enthält, ausgewählt aus einer Liste von: Toluol-2,4-diisocyanat, Diphenylmethan-2,4-diisocyanat, 1,6-Hexamethylendiisocyanat oder 10 - 20% w/v eines Polymers, ausgewählt aus folgender Liste: Polysulfon, Poly(methylacrylat), Celluloseacetat, Melamin-Formaldehyd, Polyurethan und Mischungen davon;
Herstellung einer organischen Lösung, umfassend ein flüchtiges Lösungsmittel, ausgewählt aus folgender Liste: Dichlormethan, N,N-Dimethylformamid, Aceton und Chloroform;
70 - 95 % w/v eines hydrophoben Wirkstoffs;
Rühren der organischen Lösung, insbesondere für 1 - 2 Min.;
Herstellung einer wässrigen Lösung, umfassend einen Emulgator, ein kolloidales Mittel oder Mischungen davon, wobei insbesondere der Emulgator Gummiarabikum 15 - 20% w/v, Tween 20 1 - 3 % v/v oder Mischungen davon ist und wobei das kolloidale Mittel Poly(vinylsäure) 1 - 3 % w/v ist;
Zugabe des aktiven Diffusionsmittels in die organische oder wässrige Lösung;
Erzeugung einer Öl/Wasser-Emulsion mit den vorgenannten Lösungen, bevorzugt unter mechanischem Rühren bei 400 - 1200 U/min während 3 - 8 Min.;
für die Verkapselung der hydrophoben Wirkstoffen unter Verwendung der reaktiven Monomeren Toluol-2,4-diisocyanat, Diphenylmethan-2,4-diisocyanat, 1,6-Hexamethylendiisocyanat, Zugabe eines hydrophilen Monomers zu der Emulsion, ausgewählt aus folgender Liste: Ethylendiamin, Diethylentriamin, Hexamethylendiamin, p-Phenylendiamin, 1,4-Butandiol, 1,6-Hexandiol, Ethylenglykol oder Polyethylenglykol in einem Konzentrationsbereich zwischen 0,2 und 1 mol/dm³;
für die Verkapselung der hydrophilen Wirkstoffe mit Polysulfon, Poly(methylmethacrylat), Celluloseacetat, Zugabe der Emulsion in ein Fällbad mit anschließender Verdampfung des Lösungsmittels;
Rühren der Emulsion, bevorzugt bei 400 - 800 U/min für 10 - 60 Min., bevorzugt für 40 Min.;
Sammeln der gewonnenen Nano- oder Mikrokapseln, insbesondere durch Zentrifugieren oder Filtern bei Umgebungstemperatur,
Dispergierung der gesammelten Nano- oder Mikrokapseln in wässrigen Lösungen, umfassend 10 - 20 % v/v Aminen, Polyolen, Polyethern oder Mischungen davon;
Zugabe eines Nanomaterials, wie Nanopartikel, Nanoröhren oder Nanofasern zu der Suspension der gewonnenen Nano- oder Mikrokapseln, umfassend ein photokatalytisches Material, wobei das Nanomaterial aus folgender Liste ausgewählt wird: TiO₂, WO₃, WS₂, Nb₂Os, MoO, MoS₂, V₂O₅, MgF₂, Cu₂O, NaBiO₃, NaTaO₃, SiO₂, RuO₂, BiVO₄, Bi₁₂TiO₂₀, NiO-K₄NB₆O₁₇, SrTiO₃, Sr₂NbO₇, Sr₂TaO₇,BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe oder Mischungen davon.

## Revendications

1. Capsules pour le transport d'un agent actif à propriétés photocatalytiques, ayant un diamètre externe de 0,05 - 500 µm, dans lesquelles la capsule est formée par une paroi et un noyau pour loger l'agent diffuseur
dans lesquelles la paroi de la capsule comprend un film polymère sélectionné à partir de la liste constituée de poly(méthacrylate de méthyle), mélamine-formaldéhyde, polyuréthane, polysulfone, acétate de cellulose, et des mélanges de ceux-ci,
dans lesquelles le film polymère comprend sur la surface externe des nanomatériaux, tels que des nanoparticules, nanotubes ou nanofibres chimiquement fonctionnalisés avec un composé photocatalytique sélectionné à partir d'une liste : TiO₂, WO₃, WS₂, Nb₂O₅, MoO, MoS₂, V₂O₅, MgF₂, Cu₂O, NaBiO₃, NaTaO₃, SiO₂, RuO₂, BiVO₄, Bi₁₂TiO_{2O}, NiO-K₄NB₆O₁₇, SrTiO₃, Sr₂NbO₇, Sr₂TaO₇, BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe, et des mélanges de ceux-ci,
et le noyau comprend au moins un agent actif à l'état liquide, solide ou gazeux dans lesquelles la distribution des nanomatériaux photocatalytiques sur la surface de la capsule est de 0,1 - 5% poids/volume de la capsule totale ;
dans lesquelles les nanomatériaux photocatalytiques sont chimiquement fonctionnalisés sur la surface extérieure de la paroi de la capsule.

2. Capsules selon la revendication précédente, dans lesquelles la paroi de la capsule est une distribution de film polymère comprenant de 55-80% poids/volume de la paroi totale et de nanomatériaux photocatalytiques comprenant de 20-45% poids/volume de la paroi totale.

3. Capsules selon l'une quelconque des revendications précédentes, dans lesquelles le film polymère est sélectionné à partir de la liste constituée de : poly(méthacrylate de méthyle), polysulfone, polyuréthane, ou des mélanges de ceux-ci.

4. Capsules selon l'une quelconque des revendications précédentes, dans lesquelles les capsules comprennent un film polymère de poly(méthacrylate de méthyle), et des nanomatériaux chimiquement fonctionnalisés sur leur surface extérieure comprenant un matériau photocatalytique sélectionné à partir d'une liste : TiO₂, WO₃, SrTiO₃, ZnO, ou des mélanges de ceux-ci.

5. Capsules selon l'une quelconque des revendications précédentes, dans lesquelles les nanomatériaux sous forme de nanoparticules ont un diamètre situé entre 5 et 50 nm.

6. Capsules selon l'une quelconque des revendications précédentes, dans lesquelles les nanomatériaux sous forme de nanofibres ont des longueurs allant de 10-500 nm.

7. Capsules selon l'une quelconque des revendications précédentes, dans lesquelles les nanomatériaux sous forme de nanotubes ont des diamètres de 5-100 nm, et des longueurs de 20 nm - 1 µm.

8. Capsules selon l'une quelconque des revendications précédentes, dans lesquelles l'épaisseur de la paroi de la capsule varie entre 0,05 - 25 µm; en particuler 0,2 - 10 µm.

9. Capsules selon l'une quelconque des revendications précédentes, dans lesquelles la paroi de la capsule est formée d'une pluralité de couches.

10. Capsules selon l'une quelconque des revendications précédentes, dans lesquelles le volume de l'agent actif varie entre 10⁻²⁵ - 10⁻⁵ mL, en particulier 10⁻¹⁵ - 10⁻¹⁰ mL.

11. Capsules selon l'une quelconque des revendications précédentes, dans lesquelles l'agent actif est un insectifuge, un insecticide, un agent thérapeutique, un agent de radiothérapie, un agent déodorant, une essence naturelle, une fragrance, un agent hydratant, un composant de vernis ou peinture, ou un produit agrochimique.

12. Capsules selon l'une quelconque des revendications précédentes, comprenant également au moins un tensioactif, un émulsifiant, un liant, ou des mélanges de ceux-ci ; en particulier dans lesquelles le tensioactif est sélectionné à partir de la liste suivante : hydroxyde de tétraméthylammonium, chlorure de cétrimonium, bromure de cétrimonium et chlorure de benzalkonium.

13. Articles comprenant au moins une capsule décrite dans les revendications précédentes, en particuler dans lesquels les articles sont des textiles, des fibres, du verre, du bois, du métal, des tentes, des moustiquaires, des résines, des peintures, des rideaux, des détergents, des adoucissants, des crèmes, des mousses ou des suspensions colloïdales.

14. Procédé d'obtention de capsules pour le transport d'un agent actif à propriétés photocatalytiques décrit dans l'une quelconque des revendications 1-13, comprenant les étapes suivantes :
préparation d'une solution organique comprenant 5 - 30% poids/volume d'un composé réactif sélectionné à partir de la liste suivante : 2,4-diisocyanate de toluylène, 2,4-diisocyanate de diphénylméthane, 1,6-diisocyanate d'héxaméthylène ou 10-20% poids/volume d'un polymère sélectionné à partir de la liste suivante : polysulfone, poly(méthacrylate de méthyle), acétate de cellulose, mélamine-formaldéhyde, polyuréthane, et des mélanges de ceux-ci ;
préparation d'une solution organique comprenant un solvant volatile sélectionné à partir de la liste suivante : dichlorométhane, N,N- diméthylformamide, acétone, et chloroforme ;
70-95% poids/volume d'un agent actif hydrophobique;
agitation de la solution organique, en particulier durant 1-2 min ;
préparation d'une solution acqueuse comprenant un émulsifiant, un agent colloïdal, ou des mélanges de ceux-ci, en particulier l'émulsifiant étant de la gomme arabique 15-20% poids/volume, Tween 20 1 - 3% volume/volume ou des mélanges de ceux-ci, et dans lequel l'agent colloïdal est de l'acide (poly)vinylique 1-3% poids/volume ;
ajout de l'agent diffuseur actif à la solution organique ou aqueuse ;
formation d'une émulsion d'huile/eau avec les solutions précédentes, de préférence par agitation méchanique à 400-1200 rpm durant 3-8 min ;
pour l'encapsulation d'agents actifs hydrophobiques utilisant les monomères réactifs 2,4-diisocyanate de toluylène, 2,4-diisocyanate de diphénylméthane, 1,6-diisocyanate d'héxaméthylène, ajouter à l'émulsion un monomère hydrophilique sélectionné à partir de la liste suivante : éthylènediamine, diéthylènetriamine, hexaméthylènediamine, p-phénylènediamine, 1,4-butanediol, 1,6-hexanediol, éthylène glycol ou polyéthylène glycol dans une plage de concentrations comprise entre 0,2 et 1 mol/dm³ ;
pour l'encapsulation d'agents actifs hydrophiliques utilisant du polysulfone, poly(méthacrylate de méthyle), acétate de cellulose ajouter l'émulsion à un bain de précipitation suivi par l'évaporation du solvant ;
agitation de l'émulsion, de préférence à 400-800 rpm durant 10-60 min, de préférence durant 40 min ;
collecte des nano- ou microcapsules obtenues, en particulier par centrifugation ou filtration à température ambiente,
dispersion des nano- ou microcapsules collectées dans des solutions aqueuses comprenant 10-20% volume/volume d'amines, polyols, polyéthers, ou des mélanges de ceux-ci ;
ajout à la suspension des nano- ou microcapsules obtenues d'un nanomatériau, tel qu'une nanoparticule, un nanotube, ou une nanofibre comprenant un matériau photocatalytique, dans lequel le nanomatériau est sélectionné à partir de la liste suivante : TiO₂, WO₃, WS₂, Nb₂O₅, MoO, MoS₂, V₂O₅, MgF₂, Cu₂O, NaBiO₃, NaTaO₃, SiO₂, RuO₂, BiVO₄, Bi₁₂TiO_{2O}, NiO-K₄NB₆O₁₇, SrTiO₃, Sr₂NbO₇, Sr₂TaO₇, BaTiO₃, BaTaTi₂O₅, ZnO, ZrO₂, SnO₂, ZnS, CaBi₂O₄, Fe₂O₃, Al₂O₃, Bi₂O₆, Bi₂S₃, CdS, CdSe, ou des mélanges de ceux-ci.
